# EUROPEAN PATENT APPLICATION

(11) **EP 4 835 899 A1**
(43) Date of publication of application: **04.11.2026**
(21) Application number: 26164835.6
(22) Date of filing: 13.03.2026
(51) Int. Cl.: G16B 50/40, G16B 30/00

(54) **METHOD AND SYSTEM FOR PRIVATE AND RESOURCE-EFFICIENT ABUNDANCE ESTIMATION**

(30) Priority: 29.04.2025 IN 202521041359
(71) Applicant: Tata Consultancy Services Limited, Maharashtra (IN)
(72) Inventor: SAGLANI, Divyesh, 411057 Pune, Maharashtra (IN); ALASINGARA BHATTACHAR, Rajan Mindigal, 560066 Bangalore, Karnataka (IN); SIVADASAN, Naveen, 500034 Hyderabad, Telangana (IN)
(74) Representative: Goddar, Heinz J.

(57) **Abstract**

The present disclosure addresses the problems related to k-mer abundance estimation. Conventional approaches mainly focus on efficient computations and do not address privacy concerns. The present disclosure maintains the strengths of FHE and the use of honey k-mers to provide a secure, efficient, and reliable solution for k-mer abundance estimation. The value of each honey k-mer is set to zero, ensuring that they do not alter the results of the abundance histogram. With the introduction of honey k-mers, the requirement to encrypt the sampling level and counter number is eliminated. This approach not only addresses the issues of data privacy and computational efficiency but also enhances the scalability of genomic analyses, paving the way for advancements in personalized medicine and other critical areas of research. Consequently, encrypted comparison operations are eliminated, significantly reducing the overall time complexity of the matrix update process with improved overall accuracy of the algorithm.

## Description

### CROSS-REFERENCE TO RELATED APPLICATIONS AND PRIORITY

The present application claims priority from Indian patent application no. 202521041359 filed on April 29, 2025.

### TECHNICAL FIELD

The disclosure herein generally relates to the field of cryptography and, more particularly, to a method and system for private and resource-efficient abundance estimation.

### BACKGROUND

Genomes contain genetic information within the cells of organisms. By analyzing their structural and functional formations, researchers can gain deeper understanding of genetic basis for diseases, which has potential to revolutionize healthcare, enabling personalized medicine tailored to individual genetics.

The study of genomics involves the comprehensive analysis of an organism's complete set of Deoxyribo Nucleic acid (DNA), including its genes and their functions. As genomic data continues to grow exponentially due to advancements in sequencing technologies, the need for effective computational tools and algorithms becomes increasingly important. K-mer analysis serves as a foundational technique in genomics, enabling researchers to explore genetic information, understand evolutionary relationships, and develop personalized medicine approaches. K-mers are short, contiguous sequences of nucleotides derived from a longer DNA or Ribo Nucleic Acid (RNA) sequence, where "k" represents the length of the sequence. In genomics, k-mers play a crucial role in various analyses, including genome assembly, sequence alignment, and variant detection. By breaking down genomic sequences into manageable k-mer units, researchers can efficiently analyze large datasets, identify patterns, and make inferences about genetic variations.

In the rapidly evolving field of genomics, the need for secure and efficient data processing has never been more critical. Fully Homomorphic Encryption (FHE) plays a pivotal role in this landscape, allowing researchers to perform computations on encrypted genomic data without exposing sensitive information. This capability is essential for maintaining patient privacy and complying with stringent data protection regulations, such as Health Insurance Portability and Accountability Act (HIPAA) and General Data Protection Regulation (GDPR), making FHE a cornerstone of modern bioinformatics.

Counting the occurrences of each k-mer in a genome and constructing an abundance histogram helps researchers to identify patterns and features indicative of specific biological properties. For instance, k-mer abundance histograms have been used to detect genomic rearrangements, identify viral integration sites, and characterize microbial communities. While several abundance estimation and computation algorithms exist in plain domain, most of them are resource extensive and hence not suitable to be implemented using FHE. One of the major drawbacks of FHE is the high computational cost required to implement an algorithm. Hence the traditional methods relying on complex data structures and extensive computational resources lead to inefficiencies and scalability issues in FHE.

In addressing the above said business problems related to k-mer abundance estimation, there is a significant technology gap in conventional methods, particularly in their lack of robust privacy-preserving techniques. Many conventional algorithms are vulnerable to data breaches and unauthorized access, which compromises sensitive information in fields of healthcare and bioinformatics. Additionally, as genomic data continues to grow exponentially, current solutions often struggle with scalability, leading to slower processing times and increased resource consumption.

Further, conventional approaches for k-mer abundance estimation mainly focus on efficient computations and do not address privacy concerns. A significant challenge in privacy-preserving genome analysis using Fully Homomorphic Encryption (FHE) is achieving computational efficiency due to the large size of genome data. Hence there is a challenge in resource efficient FHE based computation of abundance estimation.

### SUMMARY

Embodiments of the present disclosure present technological improvements as solutions to one or more of the above-mentioned technical problems recognized by the inventors in conventional systems. For example, in one embodiment, a method for private and resource-efficient abundance estimation is provided. The method includes receiving, by one or more hardware processors of a client machine, a raw genome sequence as an input. Further, the method includes extracting, by the one or more hardware processors of the client machine, a plurality of true k-mers from the raw genome sequence using a sketching algorithm, wherein each true k-mer is a short, fixed-length sequences of nucleotides. Furthermore, the method includes selecting, by the one or more hardware processors of the client machine, a privacy parameter alpha, wherein the privacy parameter alpha determines a plurality of honey k-mers to be included in the raw genome sequence. Furthermore, the method includes generating, by the one or more hardware processors of the client machine, a plurality of k-mers based on the plurality of true k-mers and the plurality of honey k-mers generated based on the privacy parameter alpha using a pseudo-random generator. Furthermore, the method includes computing, by the one or more hardware processors of the client machine, a row number and a column number for each of the plurality of k-mers using an associated hash value, wherein the associated hash value is generated using a random hash function. Furthermore, the method includes generating, by the one or more hardware processors of the client machine, a k-mer-collision value pair for each of the plurality of k-mers, wherein k-mer value from the k-mer-collision value pair indicates whether it is a true k-mer or a honey k-mer and a collision value from the k-mer-collision value pair indicates whether there is a hash collision. Furthermore, the method includes encrypting, by the one or more hardware processors of the client machine, the k-mer-collision value pair associated with each of the plurality of k-mers based on a Fully Homomorphic Encryption (FHE) key pair, wherein the encrypted k-mer-collision value pair is transmitted to a server machine along with unencrypted row number and unencrypted column number. Furthermore, the method includes generating, by the one or more hardware processors of the server machine, a k-mer abundance histogram for the raw genome sequence based on a sketch matrix. Finally, the method includes transmitting, by the one or more hardware processors of the server machine, the distinct k-mer count and the frequency value to the client machine, wherein the client machine receives and decrypts the distinct k-mer count and the frequency value.

In another aspect, a system for private and resource-efficient abundance estimation is provided. The system includes a client machine (102) and a server machine (104), wherein the system 100 is configured by the programmed instructions to initially receive by one or more hardware processors of a client machine, a raw genome sequence as an input. Further, the system is configured by the programmed instructions to extract, by the one or more hardware processors of the client machine, a plurality of true k-mers from the raw genome sequence using a sketching algorithm, wherein each true k-mer is a short, fixed-length sequences of nucleotides. Furthermore, the system is configured by the programmed instructions to select, by the one or more hardware processors of the client machine, a privacy parameter alpha, wherein the privacy parameter alpha determines a plurality of honey k-mers to be included in the raw genome sequence. Furthermore, the system is configured by the programmed instructions to generate, by the one or more hardware processors of the client machine, a plurality of k-mers based on the plurality of true k-mers and the plurality of honey k-mers generated based on the privacy parameter alpha using a pseudo-random generator. Furthermore, the system is configured by the programmed instructions to compute, by the one or more hardware processors of the client machine, a row number and a column number for each of the plurality of k-mers using an associated hash value, wherein the associated hash value is generated using a random hash function. Furthermore, the system is configured by the programmed instructions to generate, by the one or more hardware processors of the client machine, a k-mer-collision value pair for each of the plurality of k-mers, wherein k-mer value from the k-mer-collision value pair indicates whether it is a true k-mer or a honey k-mer and a collision value from the k-mer-collision value pair indicates whether there is a hash collision. Furthermore the system is configured by the programmed instructions to encrypt, by the one or more hardware processors of the client machine, the k-mer-collision value pair associated with each of the plurality of k-mers based on a Fully Homomorphic Encryption (FHE) key pair, wherein the encrypted k-mer-collision value pair is transmitted to a server machine along with unencrypted row number and unencrypted column number. Furthermore, the system is configured by the programmed instructions to generate, by the one or more hardware processors of the server machine, a k-mer abundance histogram for the raw genome sequence based on a sketch matrix. Finally, the system is configured by the programmed instructions to transmit, by the one or more hardware processors of the server machine, the distinct k-mer count and the frequency value to the client machine, wherein the client machine receives and decrypts the distinct k-mer count and the frequency value.

In yet another aspect, a computer program product including a non-transitory computer-readable medium embodied therein a computer program for private and resource-efficient abundance estimation is provided. The computer readable program, when executed on a client machine, causes the one or more hardware processors of a client machine, to receive a raw genome sequence as an input. Further, the computer readable program, when executed on the client machine, causes the one or more hardware processors of the client machine, to extract a plurality of true k-mers from the raw genome sequence using a sketching algorithm, wherein each true k-mer is a short, fixed-length sequences of nucleotides. Furthermore, the computer readable program, when executed on the client machine, causes the one or more hardware processors of the client machine, to select a privacy parameter alpha, wherein the privacy parameter alpha determines a plurality of honey k-mers to be included in the raw genome sequence. Furthermore, the computer readable program, when executed on the client machine, causes the one or more hardware processors of the client machine, to generate a plurality of k-mers based on the plurality of true k-mers and the plurality of honey k-mers generated based on the privacy parameter alpha using a pseudo-random generator. Furthermore, the computer readable program, when executed on the client machine, causes the one or more hardware processors of the client machine, to compute a row number and a column number for each of the plurality of k-mers using an associated hash value, wherein the associated hash value is generated using a random hash function. Furthermore, the computer readable program, when executed on the client machine, causes the one or more hardware processors of the client machine, to generate a k-mer-collision value pair for each of the plurality of k-mers, wherein k-mer value from the k-mer-collision value pair indicates whether it is a true k-mer or a honey k-mer and a collision value from the k-mer-collision value pair indicates whether there is a hash collision. Furthermore, the computer readable program, when executed on the client machine, causes the one or more hardware processors of the client machine, to encrypt the k-mer-collision value pair associated with each of the plurality of k-mers based on a Fully Homomorphic Encryption (FHE) key pair, wherein the encrypted k-mer-collision value pair is transmitted to a server machine along with unencrypted row number and unencrypted column number. Furthermore, the computer readable program, when executed on the client machine, causes the one or more hardware processors of the server machine, to generate a k-mer abundance histogram for the raw genome sequence based on a sketch matrix. Finally, the computer readable program, when executed on the client machine, causes the one or more hardware processors of the server machine, to transmit the distinct k-mer count and the frequency value to the client machine, wherein the client machine receives and decrypts the distinct k-mer count and the frequency value.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1A is a functional block diagram of a system for private and resource-efficient abundance estimation, in accordance with some embodiments of the present disclosure.
FIG. 1B illustrates the functional block diagram of a computing device for the private and resource-efficient abundance estimation, in accordance with some embodiments of the present disclosure.
FIG. 1C illustrates an example modular block diagram of the system for private and resource-efficient abundance estimation, in accordance with some embodiments of the present disclosure.
FIG. 2A and FIG. 2B (collectively referred as FIG. 2) illustrates a flow diagram for a processor implemented method for private and resource-efficient abundance estimation, in accordance with some embodiments of the present disclosure.
FIG. 3 illustrates an example resource-efficient abundance histogram for the processor implemented method for private and resource-efficient abundance estimation, in accordance with some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

A critical part of genome analysis is Deoxyribo Nucleic acid (DNA) sequencing, which determines the precise order of nucleotides (adenine, cytosine, guanine, and thymine) in a DNA sequence. Over the years, DNA sequencing technology has advanced rapidly, generating large volume of genomic data at lower costs. However, the large volume and complexity of data poses significant challenges for downstream analysis and interpretation. k-mers are short, fixed-length sequences of nucleotides extracted from larger DNA sequences. Counting the occurrences of each k-mer in a genome and constructing an abundance histogram helps researchers identify patterns and features indicative of specific biological properties. For instance, k-mer abundance histograms have been used to detect genomic rearrangements, identify viral integration sites, and characterize microbial communities. Further, k-mers assist in estimating genome length and k-mer error rates, which helps in sequencing error rate estimation.

While several abundance estimation and computation algorithms exist in plain domain, most of them are resource extensive and hence not suitable to be implemented using Fully Homomorphic Encryption (FHE). One of the major drawbacks of FHE is the high computational cost required to implement an algorithm. Hence the traditional methods relying on complex data structures and extensive computational resources lead to inefficiencies and scalability issues in FHE.

Typically, the large number of k-mers in genomic data needs to be stored strategically in a sketch matrix for efficient storage and enabling further statistical analysis. To solve the privacy-related problem when delegating the abundance computation task to a third party, for example a server machine, FHE can be used to encrypt the sampling level and counter number corresponding to the k-mers and send them to the server to perform further computations. However, a challenge arises when updating the sketch matrix.

The sketch matrix is a data structure used to efficiently summarize and approximate large datasets. It is useful for tasks like similarity estimation, data compression and sketching algorithms. If both the row and column are encrypted using FHE, updating the sketch matrix requires comparing the encrypted row and column with the total number of rows and columns. This leads to a worst-case time complexity of *O*(*W* × *R*) comparison and addition operations and one multiplication operation to check for collisions, where *W* and *R* are the total number of rows and columns in the matrix, respectively. Since comparison is an expensive operation in FHE, this approach becomes computationally impractical for large datasets, resulting in slower processing times and increased resource consumption.

Sketching techniques like Count-Min Sketch (CMS), HyperLogLog (HLL), Bloom Filters, and Stable Distribution Sketch are efficient data structures for summarizing large datasets and enabling approximate frequency queries in data streams. CMS updates counts in a two-dimensional array using multiple hash functions, while querying retrieves the minimum count from corresponding indices. HyperLogLog estimates distinct elements, Bloom Filters test membership with possible false positives, and Stable Distribution Sketch maintains counts based on stable algorithms. The present disclosure utilizes the Count-Min Sketch to efficiently estimate the frequency of k-mers in large genomic datasets, allowing it to handle high data volumes with reduced memory usage, making it ideal for bioinformatics applications.

To address the above challenges, embodiments herein provide a method and system for private and resource-efficient abundance estimation. The present disclosure provides a unique FHE-based resource efficient k-mer abundance estimation algorithm safeguarding user privacy.

The present disclosure employs the kmerlight algorithm using FHE alongside a unique integration of honey k-mers (false genome sequence) to secure genomic data processing. Honey k-mers are strategically generated k-mers designed to induce unwanted k-mers into the sketch matrix without affecting the overall abundance estimation. The value of each honey k-mer is set to zero, ensuring that they do not alter the results of the abundance histogram. This approach allows for computations on encrypted data, ensuring that sensitive genomic information remains confidential throughout the process. By incorporating honey k-mers (false genome sequence) alongside true k-mers (actual genomic sequences), the algorithm enhances data complexity, making it more difficult for potential attackers to extract meaningful information. A privacy parameter, α, controls the ratio of honey k-mers to true k-mers, allowing users to adjust the balance between security and performance according to their needs. The algorithm employs a randomization technique, such as a coin toss mechanism, to combine these k-mers, adding an extra layer of unpredictability that helps defend against statistical attacks. The sketch matrix, which is updated only for true k-mers, serves as a compact representation of the k-mer data, enabling efficient updates and quick analysis of distinct k-mers and their multiplicities. Overall, the combination of FHE, honey k-mers, and randomization techniques makes the kmerlight algorithm robust against various statistical attacks and is invariant to permutation attacks. Even with these enhanced security measures, the algorithm operates faster than existing state-of-the-art methods to handle large genomic data.

The usage of honey k-mers introduces controlled noise into the stream of data, enhancing privacy without compromising the accuracy of abundance estimation. This dual approach of utilizing both true k-mers and honey k-mers is not only unique but also provides a buffer against potential data corruption, ensuring the integrity of the analysis.

For example, a honey k-mer has a value of 0, meaning its inclusion does not lead to any changes in the sketch matrix. Since this process is also vulnerable if executed in the plain domain , hence it is fused with FHE, where the decision to update the sketch matrix based on whether a k-mer is a honey k-mer or an actual k-mer is encrypted using FHE. This provides an additional level of security. This process does not require encrypting the row and column numbers corresponding to the k-mer, allowing the operation to simplify to a matrix update using one simple multiplication and addition operation. The multiplication operation is used to determine if there are any collisions among the k-mers due to identical hash values.

Further, the present disclosure reduces computational complexity for server's sketch matrix update process by eliminating the need to encrypt both rows and columns in the sketch matrix. Traditional algorithms often require complex data structures and extensive computational resources, leading to inefficiencies and scalability issues, especially with large genomic datasets. In contrast, the present disclosure reduces the computational complexity of matrix updates to O(1) comparison operations, allowing for faster processing times and improved scalability. Honey k-mers also serve as a buffer against potential data corruption, ensuring the integrity of abundance estimation even in the presence of sequencing errors.

While FHE is a known technology, its application in k-mer abundance estimation, particularly in genomic data, requires a deep understanding of both cryptography and bioinformatics, making the specific challenges of efficiently implementing FHE in this domain non-trivial. The concept of honey k-mers to introduce noise without affecting accuracy is a unique approach that has not been previously explored. Furthermore, the combination of FHE and honey k-mers represents a unique intersection of cryptography and genomic analysis, which is not commonly found in existing technologies. Lastly, the specific strategies employed to overcome scalability challenges in processing large genomic datasets through the use of honey k-mers and optimized matrix updates are not readily apparent to those familiar with traditional methods.

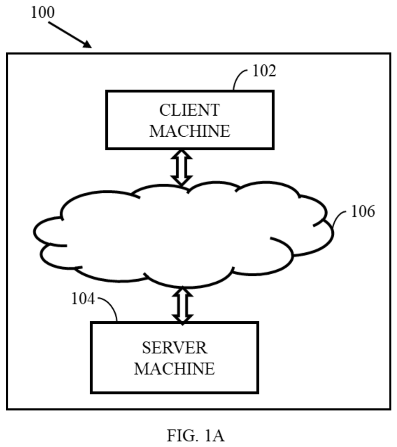

Referring now to the drawings, more particularly to FIG. 1A through FIG. 3, where similar reference characters denote corresponding features consistently throughout the figures, there are shown preferred embodiments, and these embodiments are described in the context of the following exemplary system and/or method.

FIG. 1A is an example environment system 100 for private and resource-efficient abundance estimation, according to some embodiments of the present disclosure. The system 100 includes a client 102, a server machine 104 and a network 106. The client machine 102 and the server machine 104 are connected by the network 106.

In an embodiment, the network 106 can be a wireless or a wired network, or a combination thereof. In an example, the network 106 can be implemented as a computer network, as one of the different types of networks, such as virtual private network (VPN), intranet, local area network (LAN), wide area network (WAN), the internet, and such. The network 106 may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, the network 106 may include a variety of network devices, including routers, bridges, servers, computing devices, storage devices. The network devices within the network 106 may interact with the system 102 and 104 through communication links.

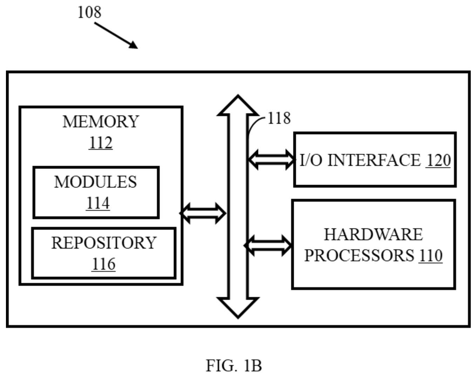

In an embodiment, the client machine 102 and the server machine 104 may be implemented in a computing device 108 as shown in FIG. 1B. The client machine 102 can be a hand-held device, a laptop or other portable computer, a tablet computer, a mobile phone, a PDA, a smartphone, and a desktop computer. The client machine 102 and the server machine 104 may also be implemented in a workstation, a mainframe computer, a server, and a network server. Similarly, the user's mobile device 106 can be any hand-held devices or mobile devices carried by a user including mobile phones and the like.

FIG. 1B is a functional block diagram of a computing device 108 associated with the system of FIG. 1A implementing the client machine 102, according to some embodiments of the present disclosure. The computing device shown in FIG. 1B is similar to the server machine 104. The computing device 108 is otherwise in communication with hardware processors 110, at least one memory such as a memory 112, an I/O interface 120. The hardware processors 110, memory 112, and the Input /Output (I/O) interface 120 may be coupled by a system bus such as a system bus 118 or a similar mechanism. In an embodiment, the hardware processors 110 can be one or more hardware processors.

The I/O interface 120 may include a variety of software and hardware interfaces, for example, a web interface, a graphical user interface, and the like. The I/O interface 120 may include a variety of software and hardware interfaces, for example, interfaces for peripheral device(s), such as a keyboard, a mouse, an external memory, a printer and the like. Further, the interface 120 may enable the access management server 104 to communicate with other devices, such the user's device 102 and the user's mobile device 106 via the network 106, web servers and external databases and the like.

The I/O interface 120 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 120 may include one or more ports for connecting a number of computing systems with one another or to another server computer. The I/O interface 120 may include one or more ports for connecting a number of devices to one another or to another server.

The one or more hardware processors 110 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 110 is configured to fetch and execute computer-readable instructions stored in memory 112.

The memory 112 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 112 includes a plurality of modules 114. The memory 112 also includes a data repository 116 for storing data processed, received, and generated by the plurality of modules 114.

The plurality of modules 114 include programs or coded instructions that supplement applications or functions performed by the access management server 104, the user's device 104 and the user's mobile device 106 for mobile device independent location based authentication. The plurality of modules 114, amongst other things, can include routines, programs, objects, components, and data structures, which performs particular tasks or implement particular abstract data types. The plurality of modules 114 may also be used as, signal processor(s), state machine(s), logic circuitries, and/or any other device or component that manipulates signals based on operational instructions. Further, the plurality of modules 114 can be used by hardware, by computer-readable instructions executed by the one or more hardware processors 110, or by a combination thereof. The plurality of modules 114 can include various sub-modules (not shown). The plurality of modules 114 may include computer-readable instructions that supplement applications or functions performed by the system 100 for mobile device independent location based authentication.

The data repository 116 may include a plurality of abstracted piece of code for refinement and data that is processed, received, or generated as a result of the execution of the plurality of modules in the module(s) 114. The data repository may also include data associated with the machine learning model used in the method for proof of decryption based biometric authentication. For example, biometric templates and biometric information associated with a plurality of users.

Although the data repository 116 is shown internal to the system 100, it will be noted that, in alternate embodiments, the data repository 116 can also be implemented external to the system 100, where the data repository 116 may be stored within a database (not shown in FIG. 1B) communicatively coupled to the system 100. The data contained within such external database may be periodically updated. For example, new data may be added into the database (not shown in FIG. 1B) and/or existing data may be modified and/or non-useful data may be deleted from the database (not shown in FIG. 1B). In one example, the data may be stored in an external system, such as a Lightweight Directory Access Protocol (LDAP) directory and a Relational Database Management System (RDBMS).

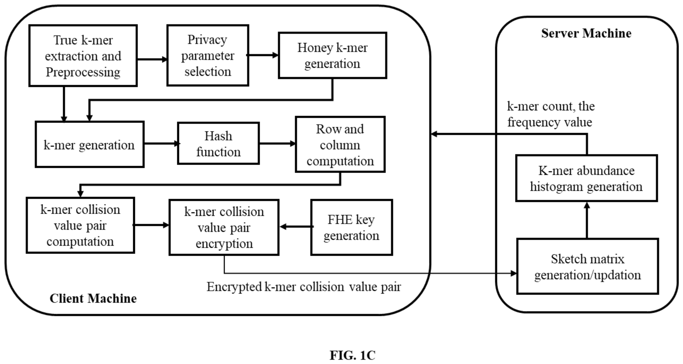

As understood by ordinary person skilled in the art, the user's device 102 has functional components similar to functional components of the access management server 104 and the user's mobile device as depicted by computing device 108 in FIG. 1B and not repeated herein for brevity. The components perform functions in accordance with instructions stored in memory block of the user's device 102 and the user's mobile device 106 enabling the devices 102 and 106 to communicate with the access management server 104. FIG. 1C illustrates an example modular block diagram of the system 100 shown in FIG. 1A for the private and resource-efficient abundance estimation, in accordance with some embodiments of the present disclosure.

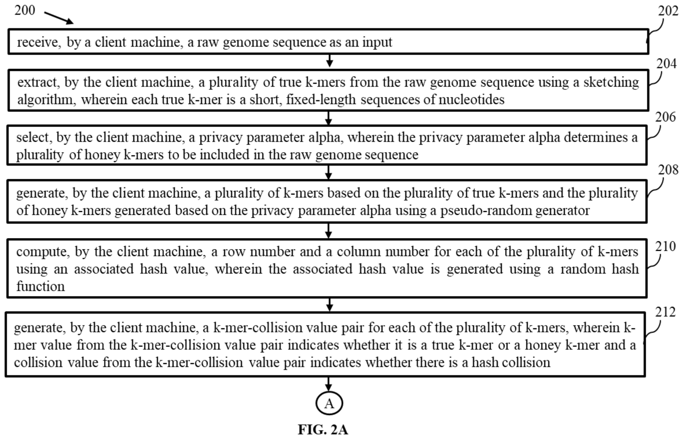

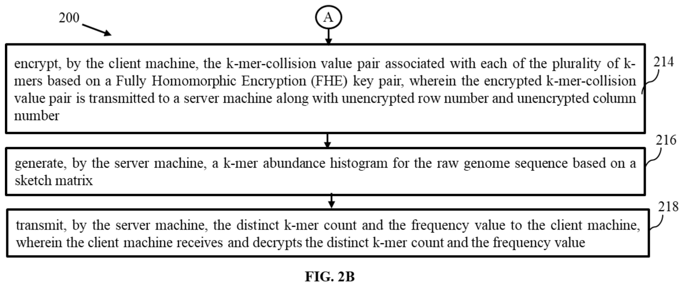

The working of the components of system 100 are explained with reference to the method steps depicted in FIG. 2, which is an exemplary flow diagram illustrating a method 200 for private and resource-efficient abundance estimation implemented by the system of FIG. 1A and 1B, according to some embodiments of the present disclosure. In an embodiment, the system 100 includes one or more data storage devices or the memory 104 operatively coupled to the one or more hardware processor(s) 102 and is configured to store instructions for execution of steps of the method 200 by the one or more hardware processors 102. The steps of method 200 of the present disclosure will now be explained with reference to the components or blocks of system 100 as depicted in FIG. 1A and 1B and the steps of flow diagram as depicted in FIG. 2. The method 200 may be described in the general context of computer executable instructions. Generally, computer executable instructions can include routines, programs, objects, components, data structures, procedures, modules, functions, etc., that perform particular functions or implement particular abstract data types.

The method 200 may also be practiced in a distributed computing environment where functions are performed by remote processing devices that are linked through a communication network. The order in which the method 200 is described is not intended to be construed as a limitation, and any number of the described method blocks can be combined in any order to implement the method 200, or an alternative method. Furthermore, the method 200 can be implemented with any suitable hardware, software, firmware, or combination thereof.

The kmerlight algorithm is a lightweight and efficient algorithm, addressing the challenges of k-mer abundance estimation in genomic sequences. It provides a perfect balance between computations and accuracy when used with FHE. Hence the present disclosure utilizes this algorithm to suggest ways to combine it with FHE for secure and resource frugal abundance estimation.

Kmerlight is a streaming algorithm, where each k-mer is mapped to a cell in a matrix *M*. To represent the k-mer, its hash is computed using a randomized hash function. This hash is used to compute a sampling level and a counter number, which represent the row and column numbers for the matrix, respectively. After updating all k-mers in the matrix, the abundance histogram is computed using the formula provided as given in equations (1) through (5): $E \left({w}_{i}\right) = \left\{\begin{matrix}{argmin}_{0 \leq w \leq W} \left|N\left(w, i\right)-R/2\left|,if i=0\right.\right. \\ {argmax}_{0 \leq w \leq W} N \left(w, i\right) , otherwise\end{matrix}\right\}$ (1) and, $E \left({t}_{i}\right) = \left\{\begin{matrix}N \left(w, i\right) - R / 2 , if i = 0 \\ N \left(w, i\right) , otherwise\end{matrix}\right\}$ (2) where, E( 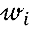 ) and E(*tᵢ*) denote the encryption of optimal row and column number in the sketch matrix and W and Rdenote total number of rows and columns in the sketch matrix respectively, which are further used to approximate k-mer counts for the histogram. $E \left({F}_{0}\right) = \left({2}^{E \left({w}_{0}\right)}\times \frac{log \left(R\right) - log \left(E\left({t}_{0}\right)\right)}{log \left(R\right) - log \left(R-1\right)}\right)$ (3) $E \left({f}_{i}\right) = \left(\frac{{2}^{E \left({w}_{i}\right)} ∗ \left(\frac{R - 1}{R}\right) ∗ E \left({t}_{i}\right)}{{\left(1-\frac{1}{R}\right)}^{{2}^{\frac{{F}_{0}}{E \left({w}_{i}\right)}}}}\right)$ (4) To reduce computation complexity of E(fi), the equation (4) is approximated as given in equation (5), $E \left({f}_{i}\right) = \frac{E \left({t}_{i}\right)}{R} \left({2}^{E \left({w}_{i}\right)}\times \left(R-1\right)+E\left({F}_{0}\right)\right)$ (5) Here, E(*F*₀) denotes the encryption of total number of distinct k-mers occurring in the genome sequence and E(fi) denotes the encryption of total number of k-mer in the genome with multiplicity *i*.

Now referring to FIG. 2, at step 202 of method 200, the one or more hardware processors 102 of a client machine, are configured by the programmed instructions to receive a raw genome sequence as an input. The raw genome sequence includes genetic information within the cells of organisms. For example, the raw genome sequence looks like "ACACAA".

At step 204 of the method 200, the one or more hardware processors 102 of the client machine, are configured by the programmed instructions to extract, a plurality of true k-mers from the raw genome sequence using a sketching algorithm, a computational technique used to create a compact representation (or "sketch") of a large dataset while preserving certain features of the original data. Each true k-mer is a short, fixed-length sequences of nucleotides. The raw genome sequence is read and preprocessed to remove any low-quality reads and contaminants during extraction. For example, for the raw genome sequence, "ACACAA", if the value of k is 3, overall there are 4, three-mers here, wiz "ACA", "CAC", "ACA", "CAA". Here, A' stands for Adenine, 'C' stands for Cytosine and 'T' stands for Thymine.

At step 206 of the method 200, the one or more hardware processors 102 of the client machine are configured by the programmed instructions to select a privacy parameter alpha (α). The privacy parameter alpha is used to determine a plurality of honey k-mers to be included in the raw genome sequence. The privacy parameter alpha (α) using the formula: *H* = *α · T,* where *T* denotes number of true k-mers, and *H* number of honey k-mers.

At step 208 of the method 200, the one or more hardware processors of the client machine 102 are configured by the programmed instructions to simultaneously generate a plurality of k-mers based on the plurality of true k-mers and the plurality of honey k-mers. The plurality of honey k-mers are generated based on the privacy parameter alpha using a pseudo-random generator.

Honey k-mers are essentially false k-mers or noise that are sent to the server alongside the actual k-mers, preventing adversaries from obtaining exact information about the k-mers being transmitted. They serve as decoy data to mislead potential attackers.

In an embodiment, the honey k-mers are strategically generated k-mers designed to induce unwanted k-mers into the sketch matrix without affecting the overall abundance estimation. The value of each honey k-mer is set to zero, ensuring that they do not alter the results of the abundance histogram. With the introduction of honey k-mers, the requirement to encrypt the sampling level and counter number is eliminated. Consequently, there is no need to perform *O*(*WxR*) encrypted comparison operations, significantly reducing the overall time complexity of the matrix update process. Additionally, by ensuring that honey k-mers do not update a sketch matrix, the present disclosure maintains the overall accuracy of the algorithm.

For example, if α = 1 then, 4 honey k-mers are generated using pseudo random generators. For example, the plurality of honey k-mers generated are "TCA", "TCG", "ACA", "CAT". In an embodiment, a true k-mer can be equivalent to a honey k-mer like "ACA".

For example, the {"ACA", "CAC", "ACA", "CAA", "TCA", "TCG", "ACA", "CAT"} be the plurality of k-mers. One of the ways to generate this plurality of k-mers is using a coin toss problem, where if head occurs, a true k-mer is chosen, else a honey k-mer is chosen. For instance, the k-mers maybe sent in this order to the server - ACA(true k-mer), CAC(honey k-mer), CAC(true k-mer), TCA(honey k-mer), TCG(honey k-mer), CAT(honey k-mer), ACA(true k-mer) and CAA(true k-mer).

At step 210 of the method 200, the one or more hardware processors 102 of the client machine is configured by the programmed instructions to compute a row number and a column number for each of the plurality of k-mers (for example, 8 k-mers) using an associated hash value. The associated hash value is generated using a random hash function. For example, the random hash functions can be a 32- bit murmur3 hash function, which can support upto 32 bit numbers.

In an embodiment, the row and the column number computed from the corresponding hash values for each k-mer using equations (6) through (9). ${r}_{i} = number of trailing zeros of {h}_{i} + 1$ (6)

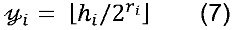

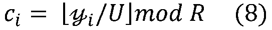

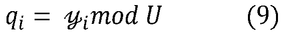

For example, for the true k-mer "ACA". The 32-bit murmur3 hash value for this is 2325258636. Considering *R* and *U* (constants) as 10 each (for simplicity), and using the equations mentioned above, the row and column number obtained are 0 and 6 respectively.

At step 212 of the method 200, the one or more hardware processors 102 of the client machine are configured by the programmed instructions to generate a k-mer-collision (v, c) value pair for each of the plurality of k-mers. The k-mer value from the k-mer-collision value pair indicates whether it is a true k-mer or a honey k-mer and a collision value from the k-mer-collision value pair indicates whether there is a hash collision. In an embodiment, considering the above mentioned example, with no collisions detected, the (v, c) value pair value for "ACA" is (1, 0). Similarly, the computations yield row number as 0, the column number as 7 and (v, c) pair value as (0,0) for the honey k-mer "CAC".

At step 214 of the method 200, the one or more hardware processors 102 of the client machine are configured by the programmed instructions to encrypt the k-mer-collision value pair (v, c) associated with each of the plurality of k-mers based on a Fully Homomorphic Encryption (FHE) key pair. The encrypted k-mer-collision value pair is transmitted to a server machine along with unencrypted row number and unencrypted column number. The FHE key pair includes a public key, a private key, an evaluation key and a bootstrapping key by defining a security level and polynomial degree. The public key is used for encryption, the private key is used for decryption, the evaluation key is used to evaluate ciphertext and the bootstrapping key is used to reduce noise in the ciphertext.

In the previous example, the pair (v, c) is encrypted using the public key generated by the client. All further operations which includes the sketch update, sketch analysis and computation of *F*₀ and *fᵢ* values are then performed using evaluation key generated following the principles of homomorphic encryption.

At step 216 of the method 200, the one or more hardware processors 102 of the server machine are configured by the programmed instructions to generate a k-mer abundance histogram for the raw genome sequence based on a sketch matrix. by: (i) Computing a distinct k-mer count based on the row number determined by finding the row number that has closest count of zeros to the half of a specified total number of columns (R) using equation (1)(ii) computing a frequency value for each multiplicity based on the row number of the sketch matrix determined by identifying the row that contains highest count of columns with a user-defined multiplicity from among a plurality of columns of the sketch matrix using equation (1). The multiplicity defines how many times a particular k-mer appears in the genome and (iii) generating the k-mer abundance histogram for the raw genome sequence based on the distinct k-mer count and the frequency value using an abundance histogram generation technique using equations (3) and (5) respectively. All these computations are performed using FHE operations, where every entry is encrypted.

The sketch matrix is generated by the server machine by multiplying the k-mer-collision value pair associated with each of the plurality of k-mers using FHE-based multiplication and further adding the result to an associated row number and an associated column number using FHE-based addition.

For example, an FHE-based encrypted sketch matrix of size *W x R* is maintained in the server machine. The next step to compute v × c using FHE multiplication. This result will be an encryption of zero if the k-mer is a honey k-mer or if there are collisions between the k-mers due to hash function. The result obtained is then added at the row and column number position sent by the client machine to the server machine. For example, referring to Table I for "ACA" k-mer the E(S[0][6]) will be set incremented by 1 using FHE addition, while for the "CAC" k-mer, the E(S[0][7]) will be incremented by 0. The next time a true k-mer ACA occurs, E(S[0][6]) is incremented by 1 again. Here S refers to the sketch matrix and E(S[i][j]) refers to the encryption of i^{th} row and j^{th} column entry of the sketch matrix.

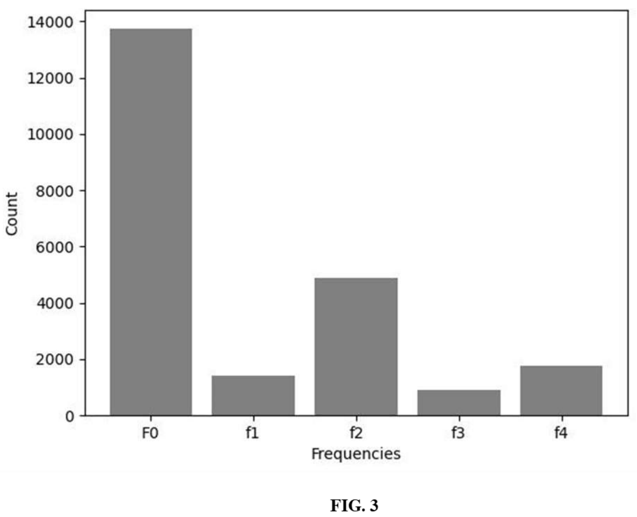

For example, the abundance histogram generated for the sketch matrix shown in Table I is given in FIG. 3. Table I shows the sketch matrix generated for the example above with *W* and *R* values as 4 and 10 respectively. Here *E*(0) denotes Encryption of 0, *E*(1) denotes encryption of 1, and so on. For E(*F*₀), the row number computed was *w*₀) = E(0*f*₁ and to compute E(*w*₁), the row number computed was E(*t*₀) = E(0)*tᵢ* The corresponding E(*t*₀) and E(t1) values were E(3) and E(1) respectively.

**Table I**

| | | | | | | | | | |
|---|---|---|---|---|---|---|---|---|---|
| E(0) | E(0) | E(0) | E(0) | E(0) | E(0) | E(2) | E(1) | E(0) | E(0) |
| E(1) | E(0) | E(0) | E(0) | E(0) | E(0) | E(0) | E(0) | E(0) | E(0) |
| E(0) | E(0) | E(0) | E(0) | E(0) | E(0) | E(0) | E(0) | E(0) | E(0) |
| E(0) | E(0) | E(0) | E(0) | E(0) | E(0) | E(0) | E(0) | E(0) | E(0) |

At step 218 of the method 200, the one or more hardware processors 102 is configured by the programmed instructions to transmit, by the server machine, the distinct k-mer count and the frequency value to the client machine, wherein the client machine receives and decrypts the distinct k-mer count and the frequency value using the private key.

### Experimentation:

The present disclosure is experimented, and the details are given below: The experiment was conducted on an AWS EC2 instance featuring an Intel(R) Xeon(R) Platinum 8275CL CPU running at 3.00GHz, along with 32 GB of RAM and 16 virtual CPUs (vCPUs). To merge true k-mers and honey k-mers, for instance one coin toss experiment is used to place honey kmer and also randomly generated a k-mer using Pseudo Random Number Generation (PRNG) techniques, where heads indicated the combination of true k-mers and tails represented the combination of honey k-mers. The value of alpha remains confidential to the client. Overall, the time to update 150,000 k-mers reduced from 64 days when both rows and columns were encrypted to 15.5 minutes using the current technology.

Security Analysis: The present disclosure seamlessly integrates honey k-mers with FHE, to secure genomic data transfer from end-to-end. In this method, both true and honey k-mers follow the same geometric distribution across the matrix, where 50% of the k-mers are mapped to the first row, the 50% of the remaining are mapped to second row, and so on. Thus, the attacker cannot rely on statistical patterns to distinguish genuine data from decoys. For example, if someone tries to reverse-engineer the sequence, they are forced to the guessing game, as every k-mer appears authentic, and the chance of correctly identifying a true k-mer becomes extremely low with the number of increases in k-mers and k value. FHE further strengthens this defense by ensuring that all k-mers, whether true or honey, remain encrypted during processing, so all computations are performed on encrypted data. Moreover, with large volume of k-mers generated, the combination of any brute-force attack or attempts to extract useful information from hash values is immense. By randomly selecting which k-mers to send to the server using a coin toss mechanism, any emerging patterns are effectively masked, leading to a very large search space of possibilities where each candidate is equally likely to be decoyed. This means that any brute-force attack or attempts to extract useful information from hash values, face large number of possibilities, reducing the risk of data breaches dramatically.

To illustrate the complexity of guessing, consider a scenario where whole genome sequence consists of 1 million true k-mers. If honey k-mers are added in ratio 1:1, the total number of k-mers doubles to 2 million. Now, assume an attacker tries to guess a specific sequence of 10 consecutive true k-mers in their correct order. Since every k-mer in the system has an equal probability of being true or honey, the chance of randomly selecting a true k-mer at any position is ½. However, since the attacker must guess 10 correct k-mers in sequence, the probability of success becomes(½)^(10) = 1/1024, which is already below 0.1%. If the honey k-mer ratio increases, e.g. alpha = 2, the probability even drops further to (1/3)^10 = 0.000016%. Furthermore because of hash based sampling and encryption, even if an attacker correctly identifies one k-mer, they still don't have a way to verify its correctness and linking to the adjacent k-mers. The large size of genome data makes it a very complex guessing problem.

Empirical performance comparisons also highlight the efficiency gains of present disclosure. In prior work, where only true k-mers were used and both row and column numbers were encrypted, the update complexity scaled poorly due to expensive encrypted comparisons for each k-mer, leading to significantly higher comparison times. An improvement on encrypting either row or column reduced the number of comparisons, increasing the efficiency of the algorithm. However, with this improvement security of overall algorithm takes a hit, as an attacker can perform statistical inference attacks to identify patterns on how k-mers are stored and updated. By introducing honey k-mers, the need for encrypted comparisons is removed while maintaining security of the overall algorithm. For example, with the introduction of honey k-mers, the requirement to encrypt the sampling level and counter number is eliminated. Consequently, there is no need to perform encrypted comparison operations, significantly reducing the overall time complexity of the matrix update process. Additionally, by ensuring that honey k-mers do not update the sketch matrix, we maintain the overall accuracy of the algorithmThe update process now operates with lower complexity leading faster execution times which are suitable for large genomic datasets.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein such computer-readable storage means contain program-code means for implementation of one or more steps of the method when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g. an ASIC and an FPGA, or at least one microprocessor and at least one memory with software modules located therein. Thus, the means can include both hardware means, and software means. The method embodiments described herein could be implemented in hardware and software. The device may also include software means. Alternatively, the embodiments may be implemented on different hardware devices, e.g., using a plurality of CPUs, GPUs and edge computing devices.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computer-usable or computer readable medium can be any apparatus that can comprise, store, communicate, propagate, or transport the program for use by or in connection with the instruction execution system, apparatus, or device. The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise. Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e. non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor-implemented method (200), the method comprising:
receiving (202), by one or more hardware processors of a client machine, a raw genome sequence as an input;
extracting (204), by the one or more hardware processors of the client machine, a plurality of true k-mers from the raw genome sequence using a sketching algorithm, wherein each true k-mer is a short, fixed-length sequences of nucleotides;
selecting (206), by the one or more hardware processors of the client machine, a privacy parameter alpha, wherein the privacy parameter alpha determines a plurality of honey k-mers to be included in the raw genome sequence;
generating (208), by the one or more hardware processors of the client machine, a plurality of k-mers based on the plurality of true k-mers and the plurality of honey k-mers generated based on the privacy parameter alpha using a pseudo-random generator;
computing (210), by the one or more hardware processors of the client machine, a row number and a column number for each of the plurality of k-mers using an associated hash value, wherein the associated hash value is generated using a random hash function;
generating (212), by the one or more hardware processors of the client machine, a k-mer-collision value pair for each of the plurality of k-mers, wherein k-mer value from the k-mer-collision value pair indicates whether it is a true k-mer or a honey k-mer and a collision value from the k-mer-collision value pair indicates whether there is a hash collision;
encrypting (214), by the one or more hardware processors of the client machine, the k-mer-collision value pair associated with each of the plurality of k-mers based on a Fully Homomorphic Encryption (FHE) key pair, wherein the encrypted k-mer-collision value pair is transmitted to a server machine along with unencrypted row number and unencrypted column number;
generating (216), by the one or more hardware processors of the server machine, a k-mer abundance histogram for the raw genome sequence based on a sketch matrix; and
transmitting (218), by the one or more hardware processors of the server machine, the distinct k-mer count and the frequency value to the client machine, wherein the client machine receives and decrypts the distinct k-mer count and the frequency value.

2. The method as claimed in claim 1, wherein the raw genome sequence is read and preprocessed to remove any low-quality reads and contaminants during extraction.

3. The method as claimed in claim 1, wherein the FHE key pair comprising a public key, a private key, an evaluation key and a bootstrapping key is generated by the client machine, wherein the public key is used for encryption, the private key is used for decryption, the evaluation key is used to evaluate ciphertext and the bootstrapping key is used to reduce noise in the ciphertext.

4. The method as claimed in claim 1, wherein the sketch matrix is generated by the server machine by multiplying the k-mer-collision value pair associated with each of the plurality of k-mers and further adding the result to an associated row number and an associated column number.

5. The method as claimed in claim 1, wherein the k-mer abundance histogram for the raw genome sequence is generated by the server machine based on the sketch matrix by:
computing a distinct k-mer count based on a lowest row number of the sketch matrix which has a maximum number of columns with value zero from among a plurality of columns of the sketch matrix;
computing a frequency value for each multiplicity based on a highest row number of the sketch matrix which has a maximum number of columns with a user-defined multiplicity from among a plurality of columns of the sketch matrix, wherein the multiplicity defines how many times a particular k-mer appears in the genome; and
generating the k-mer abundance histogram for the raw genome sequence based on the distinct k-mer count and the frequency value using an abundance histogram generation technique.

6. The method as claimed in claim 5, wherein the k-mer abundance histogram is used to detect genomic rearrangements, identify viral integration sites, characterize microbial communities and to estimate genome length and k-mer error rates.

7. A system (100) comprising:
a client machine (102) and a server machine (104), wherein the system 100 is configured by the programmed instructions to:
receive, by one or more hardware processors of a client machine, a raw genome sequence as an input;
extract, by the one or more hardware processors of the client machine, a plurality of true k-mers from the raw genome sequence using a sketching algorithm, wherein each true k-mer is a short, fixed-length sequences of nucleotides;
select, by the one or more hardware processors of the client machine, a privacy parameter alpha, wherein the privacy parameter alpha determines a plurality of honey k-mers to be included in the raw genome sequence;
generate, by the one or more hardware processors of the client machine, a plurality of k-mers based on the plurality of true k-mers and the plurality of honey k-mers generated based on the privacy parameter alpha using a pseudo-random generator;
compute, by the one or more hardware processors of the client machine, a row number and a column number for each of the plurality of k-mers using an associated hash value, wherein the associated hash value is generated using a random hash function;
generate, by the one or more hardware processors of the client machine, a k-mer-collision value pair for each of the plurality of k-mers, wherein k-mer value from the k-mer-collision value pair indicates whether it is a true k-mer or a honey k-mer and a collision value from the k-mer-collision value pair indicates whether there is a hash collision;
encrypt, by the one or more hardware processors of the client machine, the k-mer-collision value pair associated with each of the plurality of k-mers based on a Fully Homomorphic Encryption (FHE) key pair, wherein the encrypted k-mer-collision value pair is transmitted to a server machine along with unencrypted row number and unencrypted column number;
generate, by the one or more hardware processors of the server machine, a k-mer abundance histogram for the raw genome sequence based on a sketch matrix; and
transmit, by the one or more hardware processors of the server machine, the distinct k-mer count and the frequency value to the client machine, wherein the client machine receives and decrypts the distinct k-mer count and the frequency value.

8. The system as claimed in claim 7, wherein the raw genome sequence is read and preprocessed to remove any low-quality reads and contaminants during extraction.

9. The system as claimed in claim 7, wherein the FHE key pair comprising a public key, a private key, an evaluation key and a bootstrapping key is generated by the client machine, wherein the public key is used for encryption, the private key is used for decryption, the evaluation key is used to evaluate ciphertext and the bootstrapping key is used to reduce noise in the ciphertext.

10. The system as claimed in claim 7, wherein the sketch matrix is generated by the server machine by multiplying the k-mer-collision value pair associated with each of the plurality of k-mers and further adding the result to an associated row number and an associated column number.

11. The system as claimed in claim 7, wherein the k-mer abundance histogram for the raw genome sequence is generated by the server machine based on the sketch matrix by:
computing a distinct k-mer count based on a lowest row number of the sketch matrix which has a maximum number of columns with value zero from among a plurality of columns of the sketch matrix;
computing a frequency value for each multiplicity based on a highest row number of the sketch matrix which has a maximum number of columns with a user-defined multiplicity from among a plurality of columns of the sketch matrix, wherein the multiplicity defines how many times a particular k-mer appears in the genome; and
generating the k-mer abundance histogram for the raw genome sequence based on the distinct k-mer count and the frequency value using an abundance histogram generation technique.

12. The system as claimed in claim 11, wherein the k-mer abundance histogram is used to detect genomic rearrangements, identify viral integration sites, characterize microbial communities and to estimate genome length and k-mer error rates.

13. One or more non-transitory machine-readable information storage mediums comprising one or more instructions which when executed by one or more hardware processors cause:
receiving, by one or more hardware processors of a client machine, a raw genome sequence as an input;
extracting, by the one or more hardware processors of the client machine, a plurality of true k-mers from the raw genome sequence using a sketching algorithm, wherein each true k-mer is a short, fixed-length sequences of nucleotides;
selecting, by the one or more hardware processors of the client machine, a privacy parameter alpha, wherein the privacy parameter alpha determines a plurality of honey k-mers to be included in the raw genome sequence;
generating, by the one or more hardware processors of the client machine, a plurality of k-mers based on the plurality of true k-mers and the plurality of honey k-mers generated based on the privacy parameter alpha using a pseudo-random generator;
computing, by the one or more hardware processors of the client machine, a row number and a column number for each of the plurality of k-mers using an associated hash value, wherein the associated hash value is generated using a random hash function;
generating, by the one or more hardware processors of the client machine, a k-mer-collision value pair for each of the plurality of k-mers, wherein k-mer value from the k-mer-collision value pair indicates whether it is a true k-mer or a honey k-mer and a collision value from the k-mer-collision value pair indicates whether there is a hash collision;
encrypting, by the one or more hardware processors of the client machine, the k-mer-collision value pair associated with each of the plurality of k-mers based on a Fully Homomorphic Encryption (FHE) key pair, wherein the encrypted k-mer-collision value pair is transmitted to a server machine along with unencrypted row number and unencrypted column number;
generating, by the one or more hardware processors of the server machine, a k-mer abundance histogram for the raw genome sequence based on a sketch matrix; and
transmitting, by the one or more hardware processors of the server machine, the distinct k-mer count and the frequency value to the client machine, wherein the client machine receives and decrypts the distinct k-mer count and the frequency value.

14. The one or more non-transitory machine-readable information storage mediums as claimed in claim 13, wherein the raw genome sequence is read and preprocessed to remove any low-quality reads and contaminants during extraction.

15. The one or more non-transitory machine-readable information storage mediums as claimed in claim 13, wherein the FHE key pair comprising a public key, a private key, an evaluation key and a bootstrapping key is generated by the client machine, wherein the public key is used for encryption, the private key is used for decryption, the evaluation key is used to evaluate ciphertext and the bootstrapping key is used to reduce noise in the ciphertext.
